# EUROPÄISCHE PATENTSCHRIFT

(11) **EP 0 853 659 B1**
(45) Veröffentlichungstag und Bekanntmachung des Hinweises auf die Patenterteilung: **10.05.2000**
(21) Anmeldenummer: 96930009.4
(22) Anmeldetag: 25.09.1996
(51) Int. Cl.: C12M 3/00, B01L 3/00, C12M 1/20

(54) **KOMPAKTZELLKULTURSCHEIBE**
COMPACT CELL CULTURE SLIDE
LAME POUR CULTURE CELLULAIRE COMPACTE

(30) Priorität: 06.10.1995 CH 282795
(43) Veröffentlichungstag der Anmeldung: 22.07.1998
(73) Patentinhaber: Microcloning CCCD AB, 553 03 Jönköping (SE)
(72) Erfinder: LARSSON, Börje, CH-8053 Zürich (CH)
(74) Vertreter: Troesch Scheidegger Werner AG
(86) Internationale Anmeldenummer: CH9600332
(87) Internationale Veröffentlichungsnummer: WO9713839

(56) Entgegenhaltungen:
- EP-A- 0 014 007
- WO-A-95/27196
- FR-A- 2 693 739

## Beschreibung

Die vorliegende Erfindung betrifft eine Anordnung von voneinander getrennten Lebensräumen von mikroskopischen Dimensionen, ein Verfahren zur Herstellung einer solchen Anordnung sowie Methoden für Untersuchungen und Messungen mit Hilfe der Anordnung.

Die in der vorliegenden Erfindung beschriebene Anordnung von mit mikroskopischer Präzision auf einer planaren Unterlage ausgelegten, voneinander getrennten Lebensräumen von mikroskopischen Dimensionen ist insbesondere geeignet zur aseptischen Kultivierung und Beobachtung von Einzelzellen und ihren monoklonalen Nachkommen wie auch zur Züchtung und zum Studium von anderen kleinen Lebensgemeinschaften, wie Bakterien-Mensch-, Tier- oder Pflanzenzellpopulationen, Gewebekulturen, Säugetier- oder Pflanzenembryonen, Insekten oder Nematoden in verschiedenen Entwicklungsstadien.

Dabei werden die Zellen oder die anderen oben erwähnten Bioproben je einzeln oder in aktuellen Kombinationen in einen auf einer flächigen, genau präparierten Unterlage, beispielsweise einer Halbleiter-, Quarz-, Glas-, Kunststoff- oder Metallunterlage, angeordneten, mit einer dünnen semi-permeablen Folie bedeckten "Weiher" eingegeben, welcher dünn ausgebildet ist und von kleinsten Dimensionen ist und welcher somit ein Kleinststerillabor bildet. In jedem solchen Weiher, der eine schachtelartige, verschliessbare Kleinstmikrokammer bildet, werden zur jeweiligen eingegebenen Kultur angepasste Konzentrationen von beispielsweise Pufferkomponenten, Sauerstoff, Nährsubstanzen, Schutzantibiotika, Wirksubstanzen, wie Pharmaka, analytisch brauchbare Reagenzien, wie beispielsweise fluoreszierende Farbstoffe usw., mittels Penetration von Kulturmedium durch die semi-permeable, verschliessbare Deckfolie eingegeben. Die Zufuhr von beispielsweise Wachstumsfaktoren oder anderen makromolekularen Medium-spezifischen Substanzen kann, abhängig von der gewählten Porengrösse der Folie, entweder vor oder nach dem Verschliessen der Kammer bzw. des Weihers gewährleistet werden. Dabei ist es vorteilhaft, beispielsweise patienteigene Seren und kostspielige BiomolekülPräparationen besser vor dem Verschliessen einzugeben, um deren Verbrauch zu minimieren und die Konzentration in den Kammern individuell kontrollieren zu können.

Die beschriebene Anordnung bietet gute Bedingungen für die individuelle Handhabung und die mikroskopische Beobachtung der dabei je entstehenden Kulturen in ihren individuellen Eigenschaften, z.B. für die kontrollierte Exposition mit physikalischen, chemischen oder anderen Agenzien und für die optische und physikalisch-chemische Verfolgung und Charakterisierung sowie für die endgültige intravitale oder postmortale Aufbewahrung. Diese Aufbewahrung kann beispielsweise durch Tiefgefrieren oder nach chemischer Fixierung erfolgen.

Beispielsweise in der Genetik, Mikrobiologie, Immunologie, Onkologie, Radiobiologie, Pharmakologie, Embryologie, Zoologie und Botanik besteht die Notwendigkeit der individuellen Züchtung von einzelnen Zellen oder Vielzellproben, um das Wachstum und die Eigenschaften von dabei je entstehenden Kulturen zu beobachten, zu dokumentieren und lebendig oder fixiert aufzubewahren. Die Erfindung umfasst deshalb zusätzlich zur erwähnten Anordnung bzw. deren Herstellung weiter die Anpassung der beschriebenen Strukturen und entsprechenden Messverfahren zur Erfassung der zahlreichen benutzbaren, biologischen Messparametern mit Hinsicht auf die Einbettung der neuen Technologie in aktuelle biomedizinische und biotechnische Forschung und Entwicklung sowie für die Bedürfnisse der vorgesehenen, wichtigen Anwendungen in klinischer Diagnostik und Therapieplanung, für das sogenannte "genetische Engineering" und angewandte Toxikologie oder Umgebungshygiene oder Pharmakologie.

Weil die Deckfolie der Weiher für Bakterien, Viren und andere biologische Kontaminanten undurchdringlich sein kann, kann man die vorhandenen Mikrokammern als zellbiologische Sterillabors verwenden. Ein Problem ergibt sich im nur begrenzten Austausch von Nährstoffen und anderen Substanzen über die Deckfolie. Mit der vorgeschlagenen Konstruktion und der winzigen Zellmasse der einzelnen Kulturen kann dieses Austauschproblem jedoch eliminiert bzw. reduziert werden.

Es ist eine Aufgabe der vorliegenden Erfindung, eine Anordnung zu schaffen, mittels welcher gleichzeitig voneinander weitgehendst unabhängig eine Vielzahl von Bioproben kultiviert und gleichzeitig auf einfachste Art und Weise beobachtet werden kann. Im Falle der monoklonalen Zellpopulationen sollen kammerartige Kleinstlabors geschaffen werden, welche speziell günstige Konditionen für Züchtung und Analyse ermöglichen. Damit soll beispielsweise beim Vorliegen genetischer Gleichförmigkeit der Vorteil geboten werden, dass aus einer Tumorprobe, umfassend einige 100'000 oder 1'000'000 Zellen, eine zuverlässige Charakterisierung einer repräsentativen Auswahl von klonierten Zellen ermöglicht wird. Die erfindungsgemässe Aufgabe sollte beispielsweise in der klinischen Onkologie durch das Schaffen von einzelnen, voneinander getrennten, individuellen Plätzen für mindestens ca. 1'000 Klonen gelöst werden, womit die Voraussetzungen geschaffen werden für die gewünschte Repräsentativität der erwähnten Auswahl. Die Anordnung soll selbstverständlich auch für die Züchtung, das Studium und das Aufbewahren von kleineren Mengen der Zellpopulationen bis zu einer einzigen Zelle in einem Kleinlabor benutzt werden können.

Weitere Aufgaben bestehen darin, die Anordnung auf einfachste Art und Weise mit den einzelnen Zellen versehen zu können und während des Kultivierens und Beobachtens des Wachtums und der Zelleigenschaften jeden unerwünschten äusseren Einfluss auf die Uniformität der Wachstumsbedingungen in den Mikrokammern bzw. in den einzelnen "Weihern" zu eliminieren.

Wiederum eine weitere Aufgabe besteht im Schaffen von geeigneten Messmethoden, um die erfindungsgemäss geschaffenen Anordnungen bzw. die darin angeordneten Zellen oder biologischen Kulturen während der Wachstumsphase zu erfassen bzw. auszuwerten.

Die erfindungsgemäss gestellten Aufgaben werden insbesondere mittels einer Anordnung gemäss dem Wortlaut nach Anspruch 1 gelöst.

Erfindungsgemäss wird eine Anordnung zum Kultivieren von Zellen vorgeschlagen, welche eine Vielzahl von Kleinstkammern bzw. "Weihern" aufweist, welche voneinander getrennt sind und z. B. wabenartig auf einer scheibenartigen, planaren Unterlage mit mikroskopischer Präzision angeordnet sind. Die Kleinstkammern sind durch eine semi-permeable Wandung bzw. Folie mit selektiver Permeabilität überdeckt, d.h. gegenüber biologischen Kontaminationen der Umgebung verschlossen. Die Kammerstruktur ist typischerweise eine sogenannte Sandwich-struktur von parallel planaren, dünnen Platten und/oder Folien in für verschiedene Anwendungszwecke gewählten Kombinationen. Dabei sind mittig in der Sandwichstruktur angeordnete Platten bzw. Folien teilweise netzartig oder wabenartig durchbrochen ausgebildet für die Bildung der Kleinstkammern. Bei der Unterlage, welche ebenfalls Teil der "Sandwichstruktur" sein kann, handelt es sich dabei beispielsweise um eine Scheibe, gebildet durch eine dünne Silizium-, Quarz-, Glas- oder Kunststoffstruktur. Der Flächendurchmesser der Unterlage kann je nach Anzahl und Grösse der darauf anzuordnenden Kleinstkammern typischerweise 5 - 20 cm betragen. Mit der Wahl einer kreisrunden Unterlage ergibt sich eine mögliche Ausführungsvariante der Anordnung, welche sich am besten mit einer im Audio- oder Videobereich verwendeten Compact Disk vergleichen lässt, welche mittels beispielsweise mikroskopischer oder spektroskopischer Analyse der Kleinstkammern abgetastet werden kann.

Insbesondere die Überdeckung von Kleinstkammern für die Zellkultivierung mit wählbarer definierter Permeabilität bzw. mit genau vorbestimmter, selektiver Permeabilität, wie dargelegt in Anspruch 1, ist im Stand der Technik nicht vorbeschrieben. Die Überdeckung soll also nicht einfach trivialerweise für Sauerstoff durchlässig sein, wie beispielsweise in der FR-2 693 739 beschrieben, sondern es sollen die verschiedensten Materialien, wie beispielsweise Flüssigkeiten oder in Lösungen gelöste niedermolekulare Komponenten, durch die Überdeckung hindurch transportiert werden können, um gezielt auf eine Zellkultivierung einwirken zu können. In der FR-2 693 739 werden bereits zu Beginn die Zellen sowie sämtliche für das Zellwachstum verantwortliche Komponenten in die Kammer eingegeben, und die Überdeckung soll lediglich sauerstoffdurchlässig sein, damit überhaupt eine Zellkultivierung ermöglicht wird.

Die beispielsweise wabenartig angeordneten Kleinstkammern können typischerweise, jedoch nicht zwingend kreisrund ausgebildet sein, mit einem Durchmesser von ca. 0,5 - 5 mm. Zwischen der die Kammergeometrie definierenden Wabenstruktur, die auch die Verteilung, Form und Grösse der Kammern bestimmt, und der scheibenartigen Unterlage kann, wenn vorteilhaft, eine dünne, transparente Folie oder eine dünne Schicht aus einem transparenten Gel ohne Zellaffinität angeordnet werden, wie beispielsweise eine Agaroseschicht. Selbstverständlich kann auch ein anderes geeignetes Material für die Ausbildung dieser Schicht verwendet werden, welches gegenüber Zellen bzw. deren Kulturen weitgehendst inert ist oder andere Eigenschaften aufweist.

Auf dem Boden jeder Kleinstkammer der Wabenstruktur bzw. auf der Unterlage bzw. auf der erwähnten Folie oder der Gelschicht aufliegend, kann eine typischerweise konzentrisch ausgebildete zellaffinitive Fläche angeordnet werden, bestehend aus einer für das Kultivieren von Zellen geeigneten dünnen, zellaffinitiven Metall- oder Molekülschicht, wie beispielsweise eine konzentrisch ausgebildete "Palladiuminsel", oder aus einem anderen funktionsgleichen, flächenbildenden Material. Die Wabenstruktur selbst bzw. die Kammerwandungen können aus einem dimensionsstabilen Material, wie beispielsweise Kunststoff, Metall, Keramik oder einem Verbundwerkstoff gefertigt sein. Die semi-permeable Membran schlussendlich besteht vorteilhafterweise aus einem wenigstens nahezu für Licht transparenten Material, wie beispielsweise Teflon, Polycarbonat oder Polystyrol.

Weitere bevorzugte Ausführungsvarianten der erfindungsgemässen Anordnung und Verfahren zum Herstellen der Anordnungen zum Kultivieren von Zellen sind in den Ansprüchen 2 bis 11 charakterisiert.

Auf die einzelnen Verfahrensschritte zur Herstellung der erfindungsgemässen Anordnung wird unter Bezug auf die nachfolgend beschriebenen Figuren näher eingegangen. Bevorzugte Ausführungsvarianten des erfindungsgemässen Verfahrens sind in den abhängigen Ansprüchen 12 bis 16 charakterisiert.

Die vorliegende Erfindung bzw. Anordnung, ebenfalls genannt Kompaktzellkulturscheibe (Compact Cell Culture Disk oder CCCD), erlaubt mikroskopische, molekularbiologische, biochemische und physikalisch-chemische Studien von individuellen Zellen bzw. Zellen oder mehrzelligen Strukturen, sowohl in kleiner wie in grosser Anzahl, d.h. von einer einzigen Zelle bis zu typischerweise einigen 1'000 Zellen unter physikalisch chemisch oder biologisch optimierten Bedingungen. Zusammen mit biologischen Materialien in die vorgeschlagene Zellkultivierungsanordnung implantiert, entsteht ein Mikrolaboratorium, das die Steuerung, Beobachtung und Analyse der individuellen Kulturen bzw. deren Bildung zulässt, wobei die Steuerung der Milieus getrennt oder gesamthaft möglich ist. Sowohl die ganze Anordnung wie auch die einzelnen Kammern bzw. Kleinstmikrolabors können unter sterilen oder nichtsterilen Bedingungen gehalten werden, sowohl kurzzeitig wie auch für Langzeit- wie auch permanente Lagerung, den Transport oder zu Beobachtungszwecken, beispielsweise unter einem Mikroskop oder mit spektroskopischen Methoden, beispielsweise durch Ausnützung von Lichtleitern, Fotomultiplikatoren oder Fotodioden.

Schlussendlich vorgeschlagen wird ein Verfahren zum Messen bzw. Erfassen, Auswerten und gegebenenfalls Abspeichern von Informationen betreffend die in der erfindungsgemäss definierten Anordnung implantierten und gelagerten Zellen bzw. Zellkulturen-Klonen und andere, oben erwähnte Bioproben. Es handelt sich dabei vorzugsweise um automatische, EDV-unterstützte Verfahren, die die Implantations- und Analysekapazität sowie die Geschwindigkeit erhöhen können, in Verbindung mit manueller und visueller Technik.

Die Erfindung wird nun anschliessend beispielsweise und unter Bezug auf die beigefügten Figuren näher erläutert.

Dabei zeigen:
- Fig. 1: ein Beispiel einer erfindungsgemässen, scheibenartigen Anordnung in Obendraufsicht,
- Fig. 1a,b: vergrösserte Ausschnitte aus der Anordnung von Fig. 1,
- Fig 2: schematisch und in Vergrösserung verschiedene typischerweise verwendete individuelle Elemente bzw. Komponenten einer erfindungsgemässen, sandwichartigen Anordnung im Querschnitt,
- Fig. 2a: im Querschnitt eine mögliche Ausführungsvariante einer Anordnung im zusammengesetzten sowie im explosionsartig auseinandergezogenen Zustand,
- Fig. 2b: eine weitere Ausführungsvariante einer erfindungsgemässen Anordnung im Querschnitt, sowohl zusammengesetzt sowie in explosionsartig auseinandergezogener Darstellung,
- Fig. 2c: erneut eine weitere mögliche Ausführungsvariante einer erfindungsgemässen Anordnung im Querschnitt, sowohl zusammengesetzt sowie in explosionsartig auseinandergezogener Darstellung,
- Fig. 3a: schematisch dargestellt, eine mögliche in situ-Messmethode an einer erfindungsgemässen Anordnung im Querschnitt,
- Fig. 3b: schematisch dargestellt, eine weitere Messmethode von abgetötetem Material und mit abgedeckter Anordnung im Querschnitt,
- Fig. 4a: schematisch dargestellt im Querschnitt, eine Ausführungsvariante der erfindungsgemässen Anordnung,
- Fig. 4b: schematisch dargestellt im Querschnitt, eine weitere Ausführungsvariante einer erfindungsgemässen, durch Magnetfelder zusammengehaltenen Anordnung,
- Fig. 5a: in Obendraufsicht schematisch dargestellt eine erfindungsgemässe, scheibenartige, kreisrund ausgebildete Anordnung,
- Fig. 5b: eine weitere Ausführungsvariante in Obendraufsicht, analog einer Compact Disk ausgebildet, und
- Fig. 6: schematisch in Perspektive dargestellt, eine mögliche Anwendung der erfindungsgemässen Anordnung zum Untersuchen der biologischen Wirksamkeit verschiedener Strahlenarten in einem exponierten Körper als Funktion der Koordinaten in einem Probensystem.

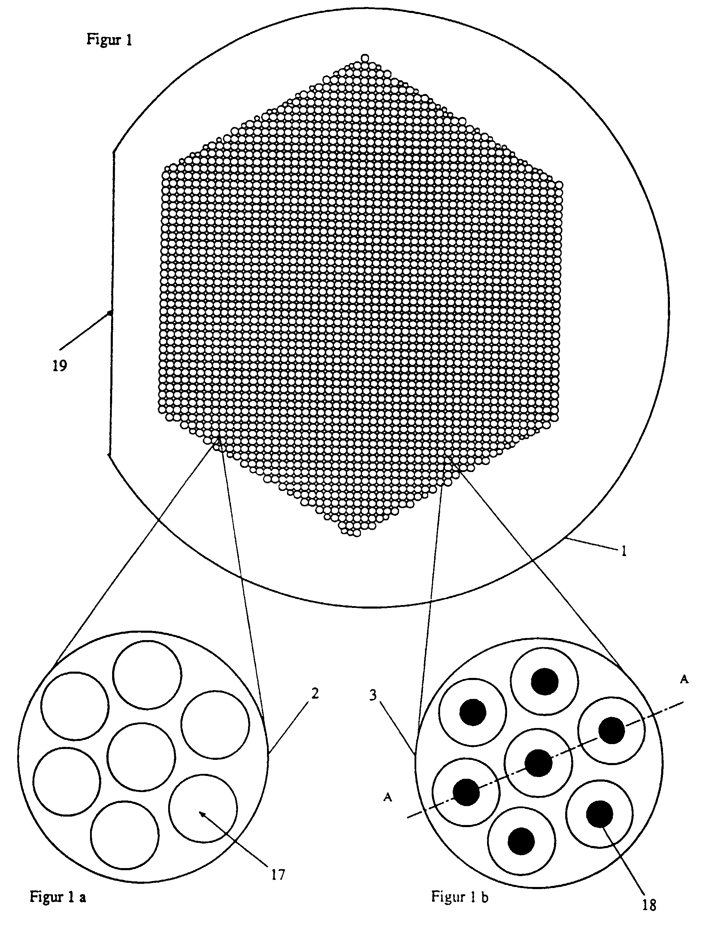

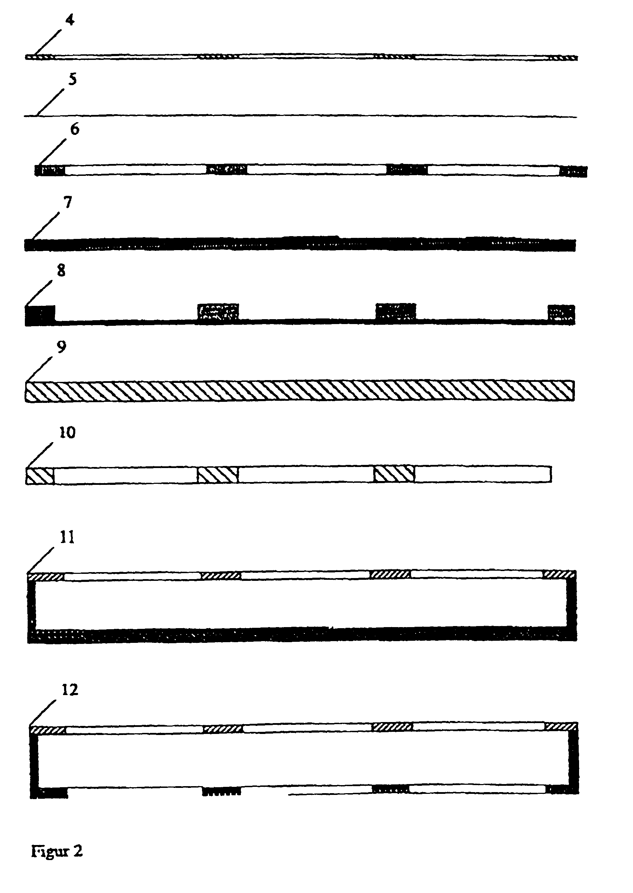

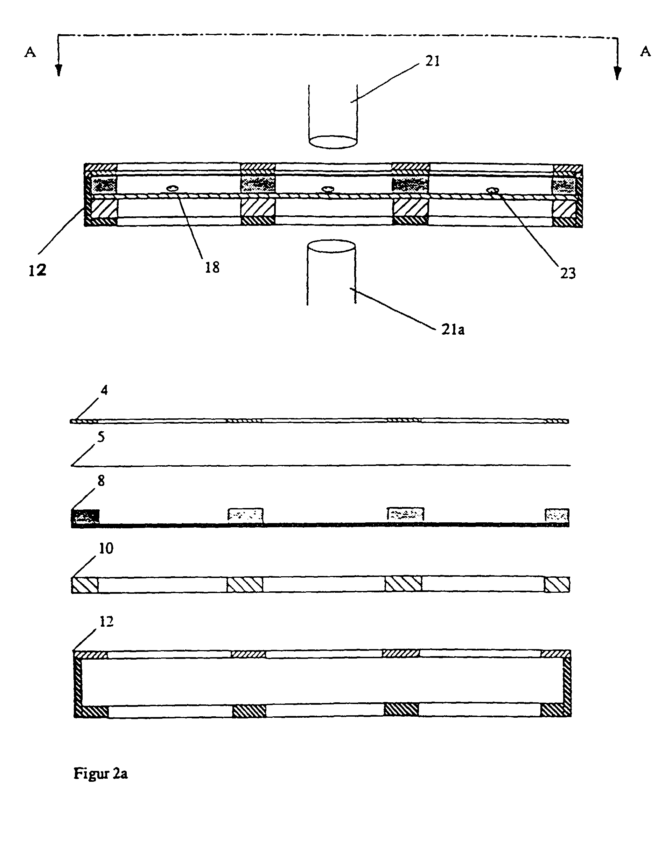

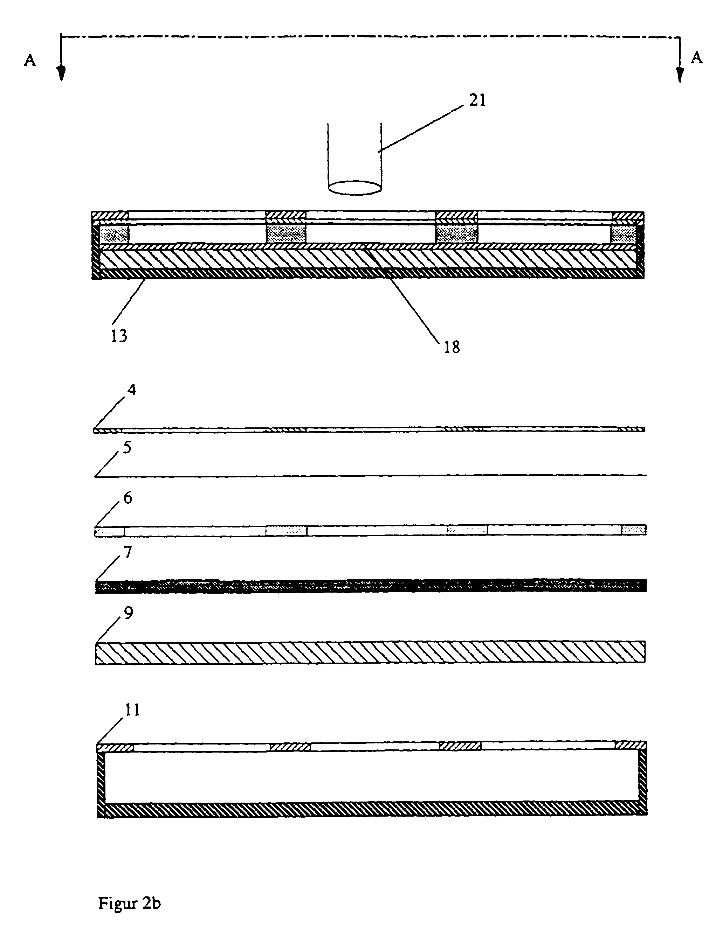

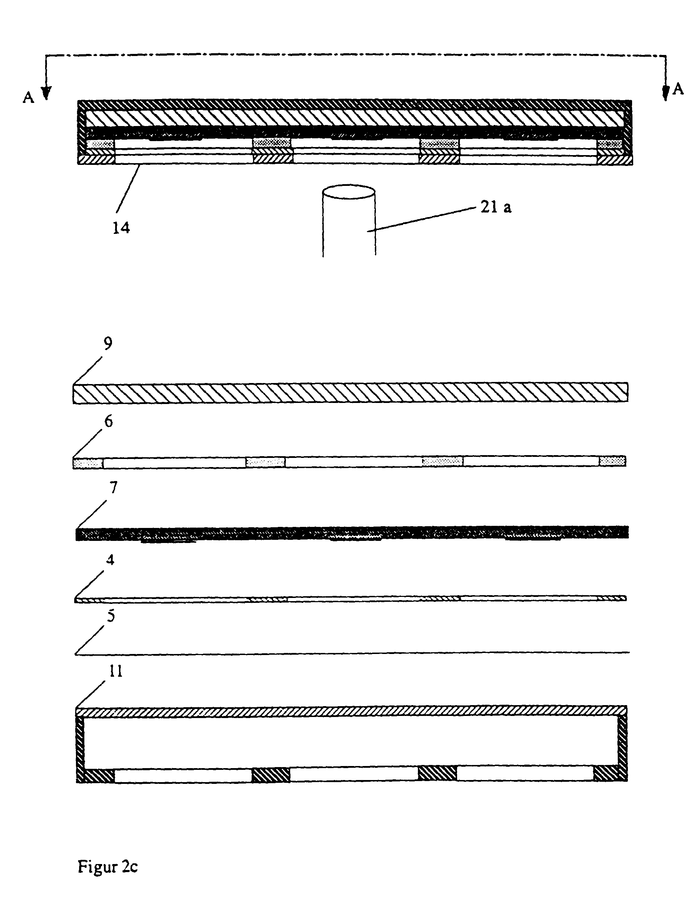

Fig. 1 zeigt in Obendraufsicht ein Beispiel einer "Compact Cell Culture Disk", zu deutsch einer Kompaktzellkulturscheibe bzw. -platte mit Kleinstkammern in zwei Ausführungsvarianten, dargestellt in den Fig. 1a und 1b. Der Aufbau dieser Scheibe 1 wird unter Bezug auf die nachfolgenden Figuren 2 und folgende näher erläutert. Im Zentrumsbereich der Scheibe 1 sind die erfindungsgemäss definierten Kleinstkammern bzw. Mikrolaboratorien 17 oder 18 angeordnet, umfassend je einen nach aussen verschliessbaren, sogenannten Weiher, wobei in jeder Kleinstkammer bzw. in jedem Weiher mittig eine zu kultivierende Zelle bzw. eine Zellkultur angeordnet werden kann. Aus fertigungstechnischen Gründen schlussendlich weist die Scheibe 1 im Randbereich eine Aussparung bzw. Einkerbung 19 auf, deren Sinn sich ebenfalls aus der nachfolgenden Beschreibung ergibt.

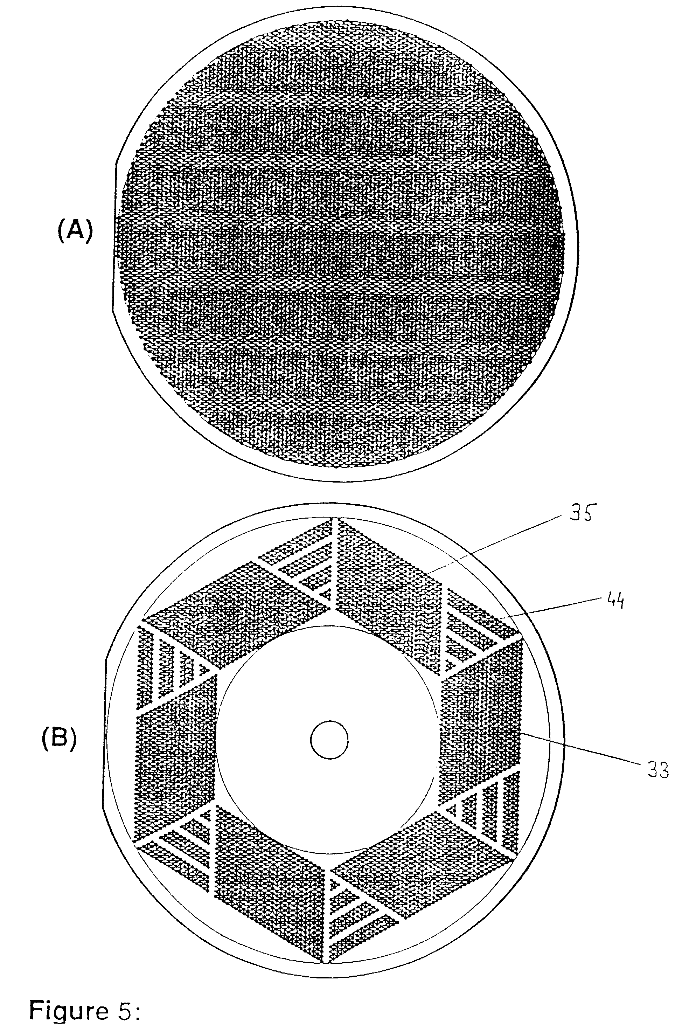

Die in Fig. 1 dargestellte Anordnung der Kleinstkammern 17 stellt nur ein Beispiel dar, und es ist selbstverständlich möglich, die Kleinstkammern 11 auf eine x-beliebige andere Art und Weise auf der Scheibe 1 anzuordnen, wie sich dies auch aus den nachfolgend beschriebenen Figuren 5a und 5b ergibt.

Ausschnittsweise aus Scheibe 1 von Fig. 1 sind in den beiden Fig. 1a und 1b je eine Anzahl Kleinstkammern 17 dargestellt, wobei in den Kleinstkammern in Fig. 1b im Zentrum zellaffinitive Flächen 18 vorgesehen sind, welche in den Kleinstkammern gemäss Fig. 1a fehlen. Solche Flächen von verschiedener Art, Lage und Ausführung und an verschiedenen Positionen oder andere zellaffinitive Strukturen, wie frei schwebende Partikel, können ebenfalls verwendet werden.

Unter Bezug auf die Fig. 2 sowie 2a bis 2c sind im Querschnitt verschiedene Ausführungsvarianten von möglichen erfindungsgemässen Anordnungen dargestellt, wobei Fig. 2 schematisch die für den Aufbau der beschriebenen Anordnungen designierten Komponenten bzw. Einzelelemente zeigt. Für den Aufbau der Anordnungen vorzusehen sind, wie in Fig. 2 dargestellt, beispielsweise eine wabenartig bzw. lochartig durchbrochen ausgebildete Metall-, Keramik- oder Kunststoffolie, sowie eine semi-permeable Wandung 5, welche beispielsweise aus Teflon, Polystyrol oder einem anderen geeigneten, vorzugsweise für Licht transparenten Material bestehen kann. Für die Bildung der eigentlichen Kleinstkammern bzw. Mikrolaboratorien wird eine gitter- oder wabenartige Matrize 6 verwendet, welche aus einem dimensionsstabilen Material gebildet wird, wie beispielsweise Kunststoff, Metall, Keramik oder einem Verbundwerkstoff bzw. verstärktem Duromeren. Diese Matrize kann aber auch aus einer mittels Oxidation oder Aetzung behandelten Siliziumscheibe gefertigt sein, wie beispielsweise in Fig. 2 mit dem Bezugszeichen 8 bezeichnet. In diesem Falle wird die oxidierte oder geätzte Siliziummatrize vorzugsweise mit einer durchsichtigen Quarzschicht angeordnet.

Als Unterlage für die Matrize 6 kann beispielsweise eine Siliziumscheibe 7 verwendet werden, welche mit zellaffinitiven Flächen 18 versehen ist, auf welchen die zu kultivierenden Zellen angeordnet werden können. Wiederum kann diese Unterlage Teil einer Siliziumscheibe sein, die mittels Oxidation und Aetzung behandelt ist, wie beispielsweise die bereits oben erwähnte Scheibe in Fig. 2 mit Bezugszeichen 8. Diese Unterlage 7 kann aber anstelle von Silizium auch aus Quarz oder Glas hergestellt sein oder aus einem anderen geeigneten Material. Vorzugsweise ist diese Siliziumunterlage mit einer dünnen Agarosepolymerschicht überdeckt, auf welcher AgaroseSchicht aufliegend die zellaffinitiven Flecken 18 angeordnet sind, beispielsweise bestehend aus Palladium. Im Gegensatz zu Palladium weist Agarose keine Zellaffinität auf. Die erwähnten Palladiumflecken eignen sich insbesondere für das Kultivieren und Klonen von einzelnen Zellen. Auf die sogenannte Palladiuminseltechnik wird an dieser Stelle nicht näher eingegangen, da sie im einschlägigen Stand der Technik für das optimale Kultivieren von einzelnen Zellen bestens bekannt ist. Verwiesen sei lediglich auf die Literaturstelle: U. Amaldi, B. Larsson, Hadrontherapy in Oncology, Proceedings of the first international symposium on Hadrontherapy, Como, Italy, 18. - 21. October 1993, Excerpta Medica, International Congress Series 1077, 1994, Elsevier, Seiten 735 ff. Selbstverständlich können auch andere ähnliche Verfahren für diese Zwecke benutzt werden.

Je nach gewählter Anordnung ist es möglich, die Sandwichkonstruktion magnetisch zusammenzuhalten, weshalb die Verwendung einer Magnetscheibe 9 vorzusehen ist. Diese Magnetscheibe kann, wie mit Bezugszeichen 9 dargestellt, flächig ausgebildet sein oder aber wabenartig bzw. lochartig durchbrochen, wie mit Bezugszeichen 10. Letztere Magnetscheibe ist dann notwendig, wenn beispielsweise durch diese Magnetscheibe hindurch optische Analytik betrieben wird.

Für das sichere Lagern, Handhaben und den Transport der sandwichartigen, erfindungsgemässen Anordnung bzw. Analysestruktur ist es vorteilhaft, ein Behältnis 11 vorzusehen, welches beispielsweise eine Edelstahlbox sein kann mit mindestens einseitig vorgesehenen Beobachtungsfenstern.

In Fig. 2a nun ist ein Behälter 11, beinhaltend eine Anordnung 12, im Querschnitt dargestellt. Zum besseren Verständnis ist die Anordnung 12 explosionsartig auseinandergezogen, ebenfalls in Fig. 2a dargestellt, womit die einzelnen, aus Fig. 2 verwendeten Komponenten sichtbar werden. Die Struktur bzw. Anordnung 12 entspricht dabei dem Schnitt entlang der Linie A-A aus Fig. 1b.

Die Struktur bzw. Anordnung 12 besteht primär aus einer wabenartigen bzw. gelochten Siliziumscheibe, welche mit einer Quarzfolie bzw. Quarzscheibe 8 kombiniert werden kann. Die lochartigen Oeffnungen der Siliziumscheibe 8 können durch Oxidation oder Aetzung aus einer Siliziumscheibe erhalten werden. Die Quarzoberfläche ist vorzugsweise mit einer Agarosepolymerschicht überdeckt, welches Material keine Zellaffinität aufweist. Die Struktur gemäss Fig. 2a umfasst weiter eine die Waben überdeckende Membran 5 sowie eine erneut wabenartig ausgebildete Nickelfolie 4, wobei die Wabenstruktur deckungsgleich ist zu derjenigen der Siliziumscheibe 8. Um die erfindungsgemässe Struktur bzw. Anordnung zusammenzuhalten wird entgegensetzt zur Siliziumwabe an der Quarzschicht eine Magnetscheibe 10 angeordnet, welche wiederum deckungsgleich zur Siliziumwabe mit entsprechenden Oeffnungen versehen ist. Durch das Anordnen der Metallfolie und der Magnetscheibe wird infolge der Magnetkräfte die Anordnung zusammengehalten. Zur sicheren Lagerung schlussendlich wird die Anordnung 12 in einem Behältnis 11, zum Beispiel aus Edelstahl, eingeführt und darin gelagert. Dabei kann diese Edelstahlbox einseitig oder beidseitig mit fensterartigen Oeffnungen versehen sein, welche wiederum deckungsgleich sind zur Siliziumwabenstruktur. Diese fensterartigen Oeffnungen dienen dazu, dass die Anordnung bzw. Struktur bzw. die darin angeordneten Zellen bzw. Zellkulturen durch geeignete Analysegeräte 21 bzw. 21a analysiert bzw. beobachtet werden können. Wie insbesondere in der zusammengesetzten Struktur dargestellt, weisen als Beispiel einer zellaffinitiven Struktur die einzelnen Kammern auf der Quarzoberfläche im Zentrum eine Palladiuminsel 18 auf.

Auf diese Palladiuminsel kann sich eine einzelne Zelle 23 anhaften, worauf die Kultivierung der Zelle einsetzt. Die einzelnen Kammern sind mit verschiedenen Flüssigkeiten, wie Seren, Nährsubstanzen, Wirksubstanzen usw. versetzt. Da die Struktur mittels der semi-permeablen Wandung 5 überdeckt bzw. verschlossen ist, ist es möglich, während des Kultivierungsvorganges weitere Substanzen in die Kammern einzugeben. Diese semi-permeable Wandung kann beispielsweise aus Teflon, Polystyrol oder einem anderen geeigneten transparenten Material sein.

In Fig. 2b ist erneut, sowohl zusammengesetzt wie auch explosionsartig auseinandergezogen, eine weitere, erfindungsgemässe Anordnung schematisch im Querschnitt und ausschnittsweise dargestellt. Wiederum entspricht der Ausschnitt in etwa dem Schnitt A-A aus Fig. 1b, wobei jedoch in Fig. 2b keine in die Struktur eingegebenen Zellen dargestellt sind. In der Anordnung bzw. Struktur gemäss Fig. 2b wird auf eine Siliziumscheibe 7 eine Matrize 6 aufgelegt, beispielsweise bestehend aus Polycarbonat. Wiederum wird die Matrize durch eine Membran 5 überdeckt, und die ganze Anordnung wird durch eine Metallfolie und Magnetscheibe mittels Magnetkräften zusammengehalten. Für die Lagerung der Anordnung ist erneut ein Behältnis 11 vorgesehen, wobei jedoch im Beispiel gemäss Fig. 2b die Analytik von oben durch den Deckel des Behältnisses 11 vorgesehen ist, mittels beispielsweise eines Messgerätes 21. Im vorliegenden Beispiel ist es vorteilhaft, wenn erneut die Siliziumscheibe mittels einer Agaroseschicht versehen ist. Wiederum auf der Agaroseschicht sind Palladiuminseln 18 vorgesehen für das Eingeben der entsprechenden Zellen.

Selbstverständlich ist es möglich, sowohl bei Struktur 12 gemäss Fig. 2a wie auch bei Struktur 13 gemäss Fig. 2b stark unterschiedliche Dimensionierungen bei der Ausgestaltung der einzelnen Mikrokammern zu wählen, doch haben sich die nachfolgenden Bemassungen beispielsweise als geeignet erwiesen:
Durchmesser der Siliziumscheibe: ca. 10 - 20 cm;
Dicke der Siliziumscheibe: 0,3 - 1 mm;
Dicke der Agaroseschicht: < 10µ;
Innendurchmesser der vorzugsweise kreisrund ausgebildeten Kleinstkammern 11: ca. 0,5 - 5 mm;
Wandstärke der Wabenstruktur zwischen den einzelnen Kammern: ca. 0,2 mm;
Höhe der Wandungen der Wabenstruktur: ca. 0,1 - 2 mm;
Durchmesser der Palladiuminsel: ca. 0,3 mm,
Dicke der Polymerdeckschicht bzw. der semi-permeablen Membran, beispielsweise bestehend aus Teflon: < 10µ.

In Fig. 2c ist erneut ein Ausschnitt schematisch im Querschnitt einer weiteren möglichen Ausführungsvariante einer erfindungsgemässen Anordnung 14 dargestellt, vorgesehen beispielsweise für die Durchführung von Messungen, zum Beobachten oder für analytische Messmethoden durch den Boden des Behältnisses 11. Die Struktur besteht erneut aus einer Silizium-Quarzscheibe 8, einer Metallfolie 4 sowie einer am Boden des Behältnisses 11 angeordneten Membran 5. Ueberdeckt wird die Struktur durch eine Magnetscheibe 9, welche gleichzeitig den Deckel des Behältnisses bzw. der Edelstahlbox 11 bildet.

Die erfindungsgemäss dargestellten Kompaktzellkulturanordnungen bzw. -strukturen 12 bis 14, umfassend die in den Fig. 2a - 2c detailliert beschriebenen Kammerstrukturen, eignen sich insbesondere für das Untersuchen von Tumoren, die eine Vielzahl unterschiedlicher Zellen beinhalten. Dabei wird zunächst der Zellhaufen einer Tumorprobe (Biopsie) aufgeteilt in einzelne Zellen dispergiert. Die Praxis hat gezeigt, dass es notwendig ist, eine relativ grosse Anzahl dieser einzelnen Zellen zu untersuchen. Eine derartige Probe umfasst in der Regel ca. 100'000 bis 1'000'000 einzelne Zellen, weshalb es wünschenswert ist, mindestens einige 1'000 einer einzelnen Probe zu untersuchen, um ein signifikantes Bild der Tumorbiopsie zu erhalten. Die Auftrennung, Dispergierung einer derartigen Zellbiopsie ist bestens bekannt.

Die einzelnen Zellen, erhalten durch die erwähnte Dispergierung, werden nun in die einzelnen, noch nicht verschlossenen Weiher 17 eingegeben. Schliesslich wird die Wabenstruktur mittels der semi-permeablen Wandung 5 nach aussen hin verschlossen, und zwar auf die erwähnte Palladiuminsel 18. Nach dem Füllen und Schliessen der Kammern 17 werden in der Regel standardisierte, niedrig molekulare Wachstumskomponenten bzw. Nährstoffe via der Deckfolie eingegeben. Der Vorteil der erfindungsgemäss beschriebenen Anordnung bzw. Struktur liegt nun darin, dass sie eine Vielzahl von kompakten, biologisch sterilen Mikrolaboratorien aufweist, welche folgende Möglichkeiten eröffnet:
1. Die Implantation von einer beliebigen Anzahl einzelner zu kultivierender Zellen (aus einer Probe von mehreren 1'000) in individuelle, in sich geschlossene Kammern, welche anschliessend beobachtet und visuell sowie instrumentell analysiert werden können.
2. Morphologische oder spektroskopische Untersuchungen der Kulturen und ihrer Umgebungsmedien unter optimalen optischen Bedingungen mittels aufrechter oder invertierter, konventioneller oder konfokaler Laserskanning-Mikroskopie.
3. Die Möglichkeit des Hinzufügens von standardisierten, niedrigmolekularen Mediumkomponenten, wie Puffer, Nährstoffe, Antibiotika.
4. Eliminierung von Abfallprodukten, abgegeben durch die lebende und wachsende Zelle durch die semi-permeable Wandung hindurch.
5. Versorgen der einzelnen Kammern mit Atmungs- oder anderen Gasen, wiederum durch die semi-permeable Wandung hindurch.
6. Kontinuierliches oder temporäres Hinzufügen von niedrigmolekularen Wirksubstanzen, wie Pharmaka oder toxischer Umgebungsfaktoren.
7. Steuerung des biochemischen und, falls relevant, des mikrobiologischen Milieus, individuell für jede Kultur zum Zeitpunkt der Implantation oder zu irgendeinem Zeitpunkt mittels Mikroinjektion durch die Deckfolie hindurch.
8. Gemeinsame oder differenzierte Beeinflussung der nichtchemischen Testmodalitäten, wie ionisierte oder nichtionisierte Strahlen zu irgendeinem Zeitpunkt mittels externer Exposition.
9. Gemeinsame oder differenzierte Temperatursteuerung.
10. Sterile Bedingungen für die Kulturen während des Lagerns, Transports, Handlings, des Analysierens und des Beobachtens in nichtsteriler Umgebung.

In den Fig. 2a bis 2c ist schematisch ein Mikroskop oder anderes optisches Gerät 21 bzw. 21a dargestellt, um darzustellen, wie die Zellen in einer einzelnen Kammer während der Untersuchung beobachtet bzw. analysiert werden können. Dabei hat es sich beispielsweise gezeigt, dass gesunde Zellen sogenannte "Monolayers" bilden, die einfach mit konventioneller Mikroskopie beobachtet werden können, währenddem sogenannte transformierte Zellen ("Tumorzellen") eine "Multilayer"-Struktur bilden können, was besser mittels eines konfokalen Mikroskopes beobachtet bzw. analysiert werden kann.

Die Ausgestaltung der erfindungsgemäss definierten Kompaktzellkulturplatte basiert auf einem neuartigen Konstruktionsprinzip von Zellkulturmikrokammern und dem Anordnen derartiger Kammern, womit sowohl begrenzte wie auch grossangelegte, manuelle oder automatische Isolation, Kultivierung, Charakterisierung und Archivierung von individuellen, lebenden Zellen, Zellpopulationen oder strukturierten Systemen von Zellen, wie kleine multizellulare Organismen oder Tumor- oder Gewebeexplantate, möglich werden.

Die erfindungsgemäss beschriebene Kompaktzellkulturplatte, sandwichartig aufgebaut auf einfachen scheibenartigen Komponenten, bringt riesige Anwendungs- und Handling-Vorteile mit sich. Zum einen ist sie relativ klein und kompakt, und zum andern ist auf ihr eine sehr grosse Vielzahl von Informationen gespeichert, welche mit bester Präzision auf einfachste Art und Weise analysierbar bzw. abrufbar sind. So kann eine derartige Zellkulturplatte bzw. -scheibe analog einer Compact Disk in ein Analysegerät bzw. Bearbeitungsgerät eingegeben werden, und mittels genau festgelegten Koordinaten kann präzise eine spezifische Zellkammer bestimmt analysiert bzw. beeinflusst werden. Auf diese Art und Weise können die vielen Kleinstmikrolabors relativ schnell abgetastet werden, beliebig oder systematisch, beispielsweise durch stufenweises, radielles und azimutales Bewegen der Scheibe.

Eine Vielzahl von Anwendungen ergibt sich für die erfindungsgemäss beanspruchte Kompaktzellkulturplatte, wie beispielsweise in der Molekular- oder Zellulargenetik, in der Mikrobiologie, in der Tumorbiologie, Toxikologie, Pharmakologie und Radiobiologie. Die "Compact Cell Culture Disk" (kurz CCCD) eignet sich für automatisierte Routineanwendungen in der Biotechnologie, klinischen Medizin oder Umwelttechnologie und findet beispielsweise auch Anwendung im Bereich des Unterrichtes. Im speziellen erlaubt die stark erhöhte Kapazität, Präzision, Reproduzierbarkeit und Aseptizität sowohl in manuellen wie auch Computer-gesteuerten Verfahren das Durchführen von sogenanntem "cell cloning", d.h. Isolation, Kultivierung, Charakterisierung und dem Archivieren von einzelnen Klonen.

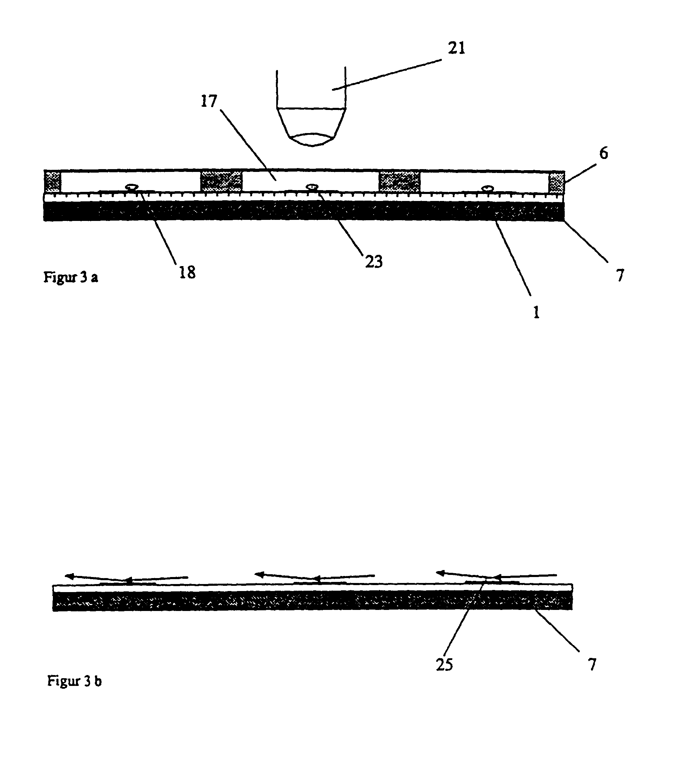

In den Fig. 3a und 3b sind schematisch zwei mögliche Mess-bzw. Analysemethoden angedeutet, gemäss welchen die zu kultivieren den bzw. die kultivierten Zell- bzw. Zellkulturen beobachtet bzw. analysiert werden können. Dabei zeigt Fig. 3a schematisch im Querschnitt eine erfindungsgemässe Anordnung 1 mit nach oben für Messungen zugängliche Kleinstkammern bzw. Laboratorien 17. Die Beobachtung bzw. Analyse der einzelnen Zellflecken 23 erfolgt somit in situ während des Kultivierungsvorganges, beispielsweise mittels eines optischen Gerätes 21, wobei selbstverständlich andere Analysegeräte verwendet werden können. Diese Messmethode ist vorteilhaft anwendbar während des Kultivierungsvorganges, da er sich beliebig wiederholen lässt. Dabei wird die gesamte Kompaktzellkulturplatte bzw. -scheibe 1 abgetastet, um eine bestimmte Auswahl oder sämtliche, in der Anordnung vorgesehene, Kammern 17 abzutasten. In Fig. 3b ist demgegenüber für abschliessendes, genaustes Messen bzw. für das Abklären des Materials die gesamte Abdeckung der ursprünglichen Struktur entfernt worden, und die Zellkulturen bzw. die kultivierten Bioproben liegen in Form toten fixierten, evt. gefriergetrockneten oder veraschten Materials 25 vor. Die Messung bzw. Analyse des abgetöteten Materials erfolgt mittels in der angedeuteten Pfeilrichtung inzidenter Photonen- oder Neutronenstrahlung. Der grosse Vorteil dieser Messmethode liegt darin, dass sie einerseits genauste Resultate vermittelt und andererseits durch das fast parallele Auftreffen der Strahlen auf das zu untersuchende Material keine Streuung in die darunterliegende Unterlage, wie beispielsweise die Siliziumscheibe, erfolgt. Messungen mittels Photonen der anderen Partikelstrahlung bzw. Röntgenstrahlung oder thermischer Neutronen ist aus dem Stand der Technik bestens bekannt.

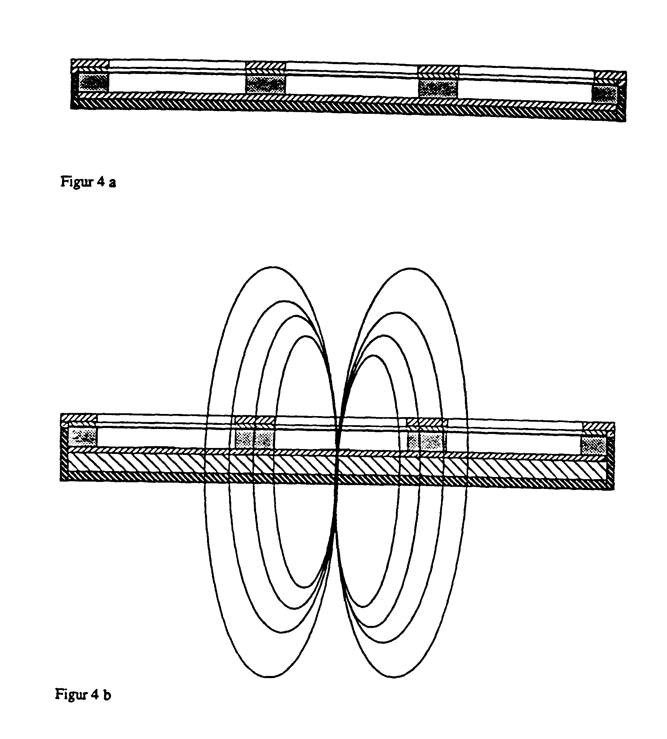

Im Gegensatz zu den in den Fig. 2a bis 2c ausschnittsweise dargestellten Kleinstkammerstrukturen bzw. Anordnungen 12 bis 14, welche sandwichartig mittels Magnetkräften zusammengehalten werden, ist es selbstverständlich auch möglich, derartige Anordnungen bzw. Strukturen auf konventionelle Art und Weise, mittels Verklebung, herzustellen. Dies ist insbesondere dann notwendig, wenn, wie in Fig. 4b im Schnitt und schematisch dargestellt, die dabei auftretenden Magnetfelder bei der Kultivierung von Zellen als störend empfunden werden. Gegebenenfalls kann es also von Nachteil sein, falls die Sandwichstruktur mittels Magnetfelder zusammengehalten wird, und es besteht deshalb gegebenenfalls die Forderung, eine erfindungsgemässe Kompaktzellkulturplatte bzw. eine Anordnung oder Struktur herzustellen, wie in Fig. 4a schematisch dargestellt, d.h. ohne dass irgendwelche Magnetfelder vorhanden sind. Eine derartige erfindungsgemässe Kompaktzellkulturplatte kann vorzugsweise entsprechend den nachfolgend beschriebenen Verfahrensschritten hergestellt werden:
1. Schaffen der wabenartigen Struktur bzw. Matrize 6 aus einem relativ steifen Material, wie beispielsweise Polycarbonat, Silizium, Keramik oder Teflon beschichtetem Nickel, mit vorzugsweise kreisrunden Lochungen, welche vorgesehen sind für die Bildung der Kleinstkammern 17. Dabei wird vorzugsweise eine möglichst hohe Packungsdichte gewählt, damit auf kleinstem Raum möglichst viele Kleinstkammern 17 ausgebildet werden können.
2. Diese gitter- oder wabenartige Struktur bzw. Matrize wird auf eine dünne Polymerfolie 5 (Membran), beispielsweise bestehend aus Teflon oder Polystyrol aufgeklebt, wobei als Klebstoff beispielsweise ein Siliconderivat oder ein UV-härtendes Polymer verwendet werden kann. Die Polymerfolie bildet die oben beschriebene, beliebig beschaffene, vorzugsweise semi-permeable Abdeckung 5 der einzelnen Kammern 17.
3. Füllen der einzelnen Weiher 17 mit Nährstoffflüssigkeiten, Seren, Wirksubstanzen usw.
4. Eingeben der einzelnen Zellen 13 in die nach oben offenen Kammern 11.
5. Auf einen Silizium-Wafer bzw. eine Glasplatte, beispielsweise aufweisend einen Durchmesser von ca. 10 cm, wird zunächst eine dünne Polymerschicht, beispielsweise bestehend aus Agarose, aufgegeben. In Uebereinstimmung mit der Wabenstruktur können auf dem Silizium-Wafer bzw. auf der Agaroseschicht die Palladiuminseln oder andere Strukturen aufgetragen oder eingegeben werden.
6. Der so hergestellte Silizium-Wafer wird auf die oben erwähnte Wabenstruktur aufgebracht, wobei vorzugsweise sowohl Wabenstruktur wie der Silizium-Wafer eine Markierung bzw. eine Einkerbung 19 (Fig. 1) aufweisen, damit der Silizium-Wafer deckungsgerecht auf der Wabenstruktur aufgebracht wird. Die Verklebung des Silizium-Wafers mit der Wabenstruktur erfolgt beispielsweise mittels Siliconfett.
7. Schlussendlich wird die so hergestellte, erfindungsgemässe Anordnung bzw. Kompaktzellkulturplatte um 180° in die Lage gemäss Fig. 3 gedreht, so dass der Silizium-Wafer als Unterlage bzw. Auflage 23 dient.

Alternativ dazu besteht die Möglichkeit, dass anstelle des Verklebens von einerseits Kunststoffolie mit der wabenartigen Struktur und anderseits der wabenartigen Struktur mit der Siliziumscheibe die bereits erwähnten Magnete verwendet werden. Zu diesem Zweck wird beim Herstellen der noch einseitig offenen Wabenstruktur gemäss Fig. 2 die Polymerfolie, bildend die semi-permeable Wand, auf eine gitterartige Nickel- oder Edelstahlfolie aufgegeben, welche in Uebereinstimmung mit den Wabenlochungen ebenfalls Lochungen aufweist. Wiederum kann je durch eine seitliche Einkerbung 19 in der Metallfolie und der Polycarbonat-Wabenstruktur sichergestellt werden, dass die einzelnen Kammern 17 durch die Nickel- oder Metallfolie hindurch, beispielsweise zu Analysezwecken, sichtbar sind. Nach Aufbringen der Siliziumscheibe 7 wird auf der Rückseite der Siliziumscheibe eine Magnetplatte 9 aufgelegt, so dass die so gebildete Anordnung bzw. Kompaktzellkultur-Sandwichstruktur mittels magnetischer Kräfte zusammengehalten wird. Schliesslich wird erneut die so gebildete Kompaktzellkulturplatte um 180° gedreht, wodurch die einzelnen Kammern 17 durch die Lochungen in der Folie hindurch zu Analyse- oder Bearbeitungszwecken sichtbar sind. Alternativ oder zusätzlich zur Magnetfolie ist es auch möglich, die wabenartige Struktur statt aus einem Polymer beispielsweise aus Nickel oder anderem magnetischem Material herzustellen.

In Fig. 5a ist ein weiteres Beispiel einer sogenannten "Compact Cell Culture Disk" dargestellt, wobei mit Ausnahme einer relativ schmalen Randpartie die ganze Platte mit der wabenartigen Struktur bzw. mit einzelnen Kleinstkammern versehen ist. Auf diese Art und Weise wird es beispielsweise möglich, bei einer Scheibe mit einem Durchmesser von 10 cm und einzelnen Kleinstkammern mit einem Durchmesser von 0,8 mm über 8'000 Kleinstkammern anzuordnen.

Fig. 5b zeigt wiederum eine weitere, erfindungsgemässe Ausgestaltung einer "Compact Cell Culture Disk", insbesondere geeignet für das Lagern, Kultivieren und Analysieren von Zellen bzw. Zellkulturen oder anderen Bioproben von unterschiedlichen Versuchsreihen. So ist es beispielsweise möglich, Zellen von einer ersten Probe in den Bereich der Struktur 33 einzugeben, währenddem Zellen von einer zweiten Probe in die einzelnen Kammern bzw. Weiher der Struktur bzw. des Bereiches 35 einzugeben sind usw. In den jeweils zwischen diesen Hauptbereichen liegenden Zwischenbereichen 34 bzw. 44 ist es möglich, Referenzzellen einzugeben, oder aber Zellen zu kultivieren und zu beobachten, für deren Kultivierung nur eine relativ kleine Anzahl notwendig ist.

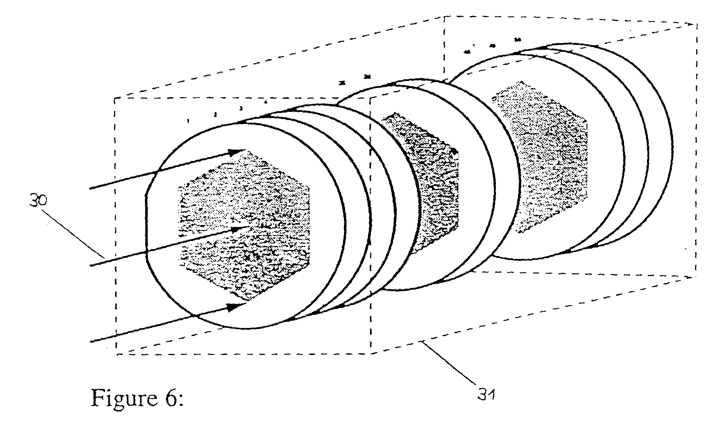

In Fig. 6 schlussendlich ist eine Anwendung der erfindungsgemäss beschriebenen Anordnungen dargestellt, insbesondere geeignet in der Strahlenforschung und Strahlenmedizin. In eine Modellstruktur 31, beispielsweise gefüllt mit Wasser, werden die einzelnen erfindungsgemäss hergestellten Kompaktzellkulturscheiben 1 eingegeben, wie in Fig. 6 dargestellt. Nun ist es möglich, die biologische Wirksamkeit in Funktion der Koordinaten der verschiedenen Probensysteme bzw. Proben auf den verschiedenen Kompaktzellkulturscheiben zu untersuchen. Dies geschieht dadurch, indem die Modellstruktur 31, beinhaltend die Kompaktzellkulturscheiben, elektromagnetischen Feldern, Röntgen-, Protonen-, Neutronen- oder x-beliebigen anderen Strahlen 30 ausgesetzt wird. Je nachdem, wo eine einzelne Kleinstkammer 17 im Koordinatensystem der Modellstruktur 31 angeordnet ist, ergibt sich eine unterschiedliche Exposition zu den durch die Struktur 31 geführten Wellen.

Bei den in den Figuren 1 - 6 dargestellten, erfindungsgemäss definierten Zellkultivierungsanordnungen, d.h. "Compact Cell Culture Disks" handelt es sich selbstverständlich nur um Beispiele, welche auf x-beliebige Art und Weise abgeändert, modifiziert und ergänzt werden können. Insbesondere ist es auch möglich, anstelle der erwähnten Bemassungen andere Dimensionen zu verwenden und anstelle der verwendeten Materialien andere geeignete zu verwenden. So ist es beispielsweise möglich, anstelle von Palladium ein anderes geeignetes Edelmetall, organische Substanzen oder chemisch fixierte Liganden zu wählen. Auch anstelle von Polycarbonat kann ein anderes geeignetes, vorzugsweise transparentes, hydrophobes Polymer, ein Metall, ein Halbleiter- oder Isolatormaterial verwendet werden. Auch als semi-permeable Deckschicht können anstelle von Teflon oder Polystyrol andere Materialien verwendet werden, wie sie allgemein für die Herstellung von vollpermeablen, semi-permeablen oder nicht permeablen Wandungen verwendet werden. Die Verwendung von Agarose auf dem Silizium-Wafer drängt sich insbesondere deshalb auf, da Agarose inert ist und von Zellen bei deren Wachstum gemieden wird. Es ist aber selbstverständlich möglich, andere Materialien für die Beschichtung des Silizium-Wafers zu verwenden, welche geeignete Eigenschaften aufweisen. Schliesslich kann anstelle der Siliziumscheibe eine durchsichtige Scheibe verwendet werden (Quarz oder ein anderes Glas) oder wiederum eine Scheibe, bestehend beispielsweise aus Polycarbonat oder einem anderen Polymeren, welche eine geringe Wasseraufnahme aufweist, wärmebeständig ist, dimensionsstabil ist und eine hohe Schlagzähigkeit aufweist. Verschiedene Kompositmaterialstrukturen sind ebenfalls aktuell, wie beispielsweise eine Wabenstruktur von Silizium mit vorgesehenen Lochungen, die mit Quarzfenstern versehen sind.

Auch in bezug auf die mögliche Anwendung der erfindungsgemäss definierten Anordnung sind keine Grenzen gesetzt. Nebst den beschriebenen Anwendungsmöglichkeiten ist es insbesondere auch möglich, derartige Kompaktzellstrukturscheiben in der Raumfahrt zu verwenden. So ist es beispielsweise möglich, Zellen bzw. Zellkulturen, angeordnet auf einer erfindungsgemäss definierten Kompaktzellkulturscheibe, Weltraumbedingungen auszusetzen, um so beispielsweise Auswirkungen von Schwerelosigkeit, von kosmischen Strahlungen usw. auf die einzelnen Zellen während deren Kultivierung zu untersuchen.

Wesentlich ist, dass bei der Wahl der verschiedenen Materialien eine Anordnung zum Kultivieren von Zellen erhalten werden kann, wodurch es möglich wird, eine Vielzahl von Kleinstkammern auf einer Unterlage anzuordnen, welche voneinander getrennt werden können, durch eine vorzugsweise semi-permeable Wandung von der Umgebung verschlossen sind und durch geeignete Fensteröffnungen beobachtet werden können.

## Patentansprüche

1. Anordnung zum Kultivieren von Zellen und/oder von anderen Bioproben, gekennzeichnet durch eine Vielzahl von Kleinstkammern, gebildet durch mit Folien überdeckten Weihern (17), welche voneinander getrennt sind und welche durch eine Wandung (5) von wählbar definierter Permeabilität von der Umgebung verschliessbar sind, wobei die Kleinstkammern oder die mit der Wandung (5) überdeckten Weiher (17) auf einer scheibenartigen, planaren, wenigstens nahezu glatten Unterlage (7) angeordnet sind, und wobei die Kleinstkammern oder Weiher durch eine Sandwich-Struktur definiert bzw. gebildet sind, aus aufeinander abgestimmten bzw. zueinander angepassten kongruenten Scheiben, Folien oder Wandungen, wobei zur Bildung der Kleinstkammern oder Weiher eine oder mehrere der Scheiben, Folien oder Wandungen eine waben- oder matrixartige Struktur mit Lochungen (17, 18) aufweisen.

2. Anordnung nach Anspruch 1, dadurch gekennzeichnet, dass die Unterlage durch eine dünne plan-parallele, vorzugsweise glatte Scheibe von optisch hoher und gleichförmiger Qualität gebildet wird.

3. Anordnung nach einem der Ansprüche 1 oder 2, dadurch gekennzeichnet, dass mindestens ein Teil der Kleinstkammern oder Weiher (17) durch eine auf einer Unterlage (7) aufliegenden und mit dieser fest verbundenen, wabenartigen Gitterstruktur oder Matrize (6) gebildet wird, wobei die der Unterlage (7) entgegengesetzten Oeffnungen der Kleinstkammern (17) oder der Gitterzwischenräume mittels einer semi-permeablen Deckmembran (5) oder Folie überdecktbar bzw. verschliessbar sind, welche Membran die Wandung mit wählbar definierter Permeabilität bildet.

4. Anordnung nach einem der Ansprüche 1 bis 3, dadurch gekennzeichnet, dass die einzelnen Kleinstkammern oder Weiher (17) kreisrund ausgebildet sind mit einem Durchmesser von ca. 0,2 - 5 mm und dass mindestens ein Teil der Kleinstkammern oder Weiher in Form einer Wabenstruktur angeordnet ist.

5. Anordnung nach einem der Ansprüche 1 bis 4, dadurch gekennzeichnet, dass die Unterlage (7) mittels einer transparenten Gel- oder Polymerschicht ohne Zellaffinität überdeckt ist, auf welcher die Kleinstkammern oder Weiher (17) angeordnet sind.

6. Anordnung nach einem der Ansprüche 1 bis 5, dadurch gekennzeichnet, dass auf dem Kammerboden, auf der Unterlage bzw. der Gel- oder Polymerschicht aufliegend, je ein punktförmig oder inselförmig ausgebildeter Fleck (18), bestehend aus einer für das Kultivieren von Zellen geeigneten dünnen Schicht, angeordnet ist.

7. Anordnung nach einem der Ansprüche 1 bis 6, dadurch gekennzeichnet, dass die Gitterstruktur bzw. die Kammerwandungen (6) aus einem steifen und dimensionsstabilen Material, wie Polycarbonat, Silizium, einem metallischen Werkstoff, Keramik oder einem Verbundwerkstoff besteht.

8. Anordnung nach einem der Ansprüche 1 bis 7, dadurch gekennzeichnet, dass die vorzugsweise semi-permeable Deckmembran (5) aus einem Polymermaterial, wie Teflon, Polystyrol oder Polycarbonat, mit wählbaren Permeabilitätseigenschaften, besteht.

9. Anordnung nach einem der Ansprüche 1 bis 8, dadurch gekennzeichnet, dass die kreisrunde Scheibe einen Durchmesser von ca. 5 - 20 cm aufweist mit einer zentralen Lochung, um die Scheibe auf einer Halterung eines Mikroskopes, Messgerätes oder Bearbeitungsgerätes anzuordnen.

10. Anordnung nach einem der Ansprüche 1 bis 9, dadurch gekennzeichnet, dass die Anordnung oder Struktur mittels einer Magnetanordnung sandwichartig zusammengehalten ist.

11. Anordnung nach einem der Ansprüche 1 bis 10, dadurch gekennzeichnet, dass die Anordnung in einem Behältnis (11) angeordnet ist, wie in einer Edelstahlbox, mit einseitig oder beidseitig für Licht durchlässige Oeffnungen, um bei im Behältnis angeordneter Anordnung Messungen durchzuführen.

12. Verfahren zur Herstellung einer Anordnung zum Kultivieren von Zellen oder anderen Bioproben, gekennzeichnet durch die Schritte:
- des Ausbildens einer gitter- oder wabenartigen Struktur (6), beinhaltend als Zwischenräume bzw. Lochungen die offenen Kleinstkammern oder Weiher als Kultivierungsräume für die einzelnen Zellen oder Bioproben, d.h. für das Bilden von Kleinstlabors (17),
- des Aufbringens und Verbindens der gitter- oder wabenartigen Struktur auf einer semi-permeablen Folie (5) oder Deckmembran,
- nach dem Füllen der einzelnen, nach unten durch die semi-permeable Folie oder Deckmembran verschlossenen Kleinstkammern oder Weiher (17) mit Nährstoffen, Seren, Wirksubstanzen oder anderen flüssigen bzw. gelösten oder lösbaren Bestandteilen sowie dem Eingeben der Zellen (23) oder Bioproben in die einzelnen Kleinstkammern oder Weiher (17), des Aufbringens einer scheibenartigen Platte (7) als Unterlage, um die Kleinstkammern bzw. Weiher entgegengesetzt zur semi-permeablen Folie oder Deckmembran (5) zu verschliessen, und
- des Drehens der gebildeten Anordnung um 180°.

13. Verfahren nach Anspruch 12, dadurch gekennzeichnet, dass die semi-permeable Wandung mit der waben- oder gitterartigen Struktur mittels eines Siliconderivates oder eines UV-härtenden Materials fest verbunden wird.

14. Verfahren nach Anspruch 12 oder 13, dadurch gekennzeichnet, dass die scheibenartige Unterlage (7) aus Silizium, Quarz oder Glas gefertigt wird, anschliessend mittels einer transparenten Gel- oder Polymerschicht ohne Zellaffinität, wie einer Agaroseschicht, beschichtet wird und schliesslich geometrisch übereinstimmend mit den Zwischenräumen bzw. Lochungen (17) der Struktur (6) mit punkt- bzw. inselförmigen zellaffinitiven Flecken (18) versehen wird, worauf anschliessend die Unterlage derart deckungsgerecht mit der Struktur verbunden wird, dass jeweils der Fleck im wesentlichen mittig im Zwischenraum oder der Lochung zu liegen kommt.

15. Verfahren nach einem der Ansprüche 12 bis 14, dadurch gekennzeichnet, dass die Unterlage und die Struktur mittels einer fettartigen Substanz, wie Siliconfett, fest miteinander verbunden werden.

16. Verfahren nach einem der Ansprüche 12 bis 14, dadurch gekennzeichnet, dass die Deckmembran (5) auf eine mit Lochungen versehene Metallplatte oder -folie aufgelegt wird, wobei die Lochungen geometrisch den Zwischenräumen bzw. Lochungen der Struktur entsprechen, und anschliessend an das Aufbringen der Struktur (6) und der Unterlage (7) ein Magnet auf die Unterlage, entgegengesetzt zur Struktur, aufgebracht wird, um infolge der Anziehung der Metallplatte oder -folie durch den Magneten die Anordnung zusammenzuhalten, worauf schlussendlich die Anordnung um 180° gedreht wird.

17. Verwendung der Anordnung nach einem der Ansprüche 1 bis 11 für die Durchführung von Messungen, dadurch gekennzeichnet, dass die Anordnung bzw. die Kammerstruktur derart zerlegt wird, dass das kultivierte Material freigelegt wird und abgetötet werden kann, worauf am abgetöteten Material Messungen mittels Photonenstrahlung oder Neutronen durchgeführt werden.

18. Verwendung der Anordnung nach einem der Ansprüche 1 bis 11 für die Durchführung von Messungen, dadurch gekennzeichnet, dass die Anordnung oder die Kammerstruktur derart zerlegt wird, dass das kultivierte Material für biochemische oder molekularbiologische Untersuchungsmethoden, wie Elektrophorese oder andere Separationsmethoden, freigelegt wird.

## Claims

1. Arrangement for culturing cells and/or other biosamples, characterized by a multiplicity of microchambers which are formed by film-covered ponds (17) which are separated from each other and which can be sealed from the environment by a wall (5) of selectably defined permeability, with the microchambers or the ponds (17), which are covered by the wall (5), being arranged on a disc-like, planar, at least almost smooth support (7), and with the microchambers or ponds being defined or formed by a sandwich structure consisting of congruent discs, films or walls which are matched to each other or fitted to each other, with, for the purpose of forming the microchambers or ponds, one or more of the discs, films or walls exhibiting a honeycomb-like or matrix-like structure having perforations (17, 18).

2. Arrangement according to Claim 1, characterized in that the support is formed by a thin, plane-parallel, preferably smooth disc of optically high and uniform quality.

3. Arrangement according to either Claim 1 or 2, characterized in that at least some of the microchambers or ponds (17) are formed by a honeycomb-like lattice structure or matrix (6) which lies on a support (7) and is firmly connected to this support, with the openings of the microchambers (17), or of the lattice gaps, which are located opposite the support (7) being coverable or sealable by means of a semipermeable covering membrane (5) or film, which membrane forms the wall having a selectably defined permeability.

4. Arrangement according to one of Claims 1 to 3, characterized in that the individual microchambers or ponds (17) are circular in form, having a diameter of approx. 0.2-5 mm, and in that at least some of the microchambers or ponds are arranged in the form of a honeycomb structure.

5. Arrangement according to one of Claims 1 to 4, characterized in that the support (7) is overlaid with a transparent gel layer or polymer layer having no cell affinity, on which layer the microchambers or ponds (17) are arranged.

6. Arrangement according to one of Claims 1 to 5, characterized in that a spot (18), which is in the form of a dot or an island and which consists of a thin layer which is suitable for culturing cells, is arranged to lie on the chamber floor, on the support or on the gel layer or polymer layer.

7. Arrangement according to one of Claims 1 to 6, characterized in that the lattice structure or the chamber walls (6) consist(s) of a rigid and dimensionally stable material, such as polycarbonate, silicon, a metallic material, ceramic or a composite material.

8. Arrangement according to one of Claims 1 to 7, characterized in that the preferably semipermeable covering membrane (5) consists of a polymer material, such as Teflon, polystyrene or polycarbonate, having selectable permeability properties.

9. Arrangement according to one of Claims 1 to 8, characterized in that the circular disc exhibits a diameter of approx. 5-20 cm having a central perforation in order to arrange the disc on a mount of a microscope, measuring instrument or processing instrument.

10. Arrangement according to one of Claims 1 to 9, characterized in that the arrangement or structure is held together, in the manner of a sandwich, by means of a magnet arrangement.

11. Arrangement according to one of Claims 1 to 10, characterized in that the arrangement is arranged in a receptacle (11), such as in a high-grade steel box having light-pervious openings on one side or both sides, in order to carry out measurements in the arrangement which is arranged in the receptacle.

12. Process for fabricating an arrangement for culturing cells or other biosamples, characterized by the following steps:
- forming a lattice-like or honeycomb-like structure (6) which comprises, as gaps or perforations, the open microchambers or ponds, as spaces for culturing the individual cells or biosamples, i.e. for forming microlaboratories (17),
- applying and connecting the lattice-like or honeycomb-like structure to a semipermeable film (5) or covering membrane,
- after filling the individual microchambers or ponds (17), which are sealed below by the semipermeable film or covering membrane, with nutrients, sera, active substances or other liquid or dissolved or dissolvable constituents, and adding the cells (23) or biosamples to the individual microchambers or ponds (17), applying a disc-like plate (7), as the support, in order to seal the microchambers or ponds on the opposite side from the semipermeable film or covering membrane (5), and
- turning the arrangement which has been formed through 180°.

13. Process according to Claim 12, characterized in that the semipermeable wall is firmly connected to the honeycomb-like or lattice-like structure by means of a silicone derivative or a UV-hardening material.

14. Process according to Claim 12 or 13, characterized in that the disc-like support (7) is manufactured from silicon, quartz or glass, is then coated with a transparent gel layer or polymer layer having no cell affinity, such as an agarose layer, and finally provided with spots (18), which are in the form of dots or islands and which have cell affinity, in a manner which coincides geometrically with the gaps or perforations (17) in the structure (6), after which the support is subsequently connected to the structure in a correctly coinciding manner such that in each case the spot essentially comes to lie dead centre in the gap or the perforation.

15. Process according to one of Claims 12 to 14, characterized in that the support and the structure are firmly connected to each other by means of a greasy substance such as silicone grease.

16. Process according to one of Claims 12 to 14, characterized in that the covering membrane (5) is laid on a metal plate or metal film which is provided with perforations, with the perforations corresponding geometrically to the gaps or perforations in the structure and, subsequent to the application of the structure (6) and the support (7), a magnet is applied to the support, on the opposite side to the structure, in order to hold the arrangement together as a result of the magnet attracting the metal plate or metal film, after which the arrangement is finally turned through 180°.

17. Use of the arrangement according to one of Claims 1 to 11 for carrying out measurements, characterized in that the arrangement or the chamber structure is disassembled in such a way that the cultured material is exposed and can be killed, after which measurements are carried out on the killed material using photon radiation or neutrons.

18. Use of the arrangement according to one of Claims 1 to 11 for carrying out measurements, characterized in that the arrangement or the chamber structure is disassembled in such a way that the culture material is exposed for biochemical or molecular biological investigation methods, such as electrophoresis or other separation methods.

## Revendications

1. Dispositif pour cultiver des cellules et/ou d'autres échantillons biologiques, caractérisé par un grand nombre de chambres miniatures formées par des viviers (17) qui sont couverts par des feuilles, qui sont séparés les uns des autres et qui sont aptes à être fermés par rapport à l'environnement par une paroi (5) d'une perméabilité apte à être choisie, étant précisé que les chambres miniatures ou les viviers (17) couverts par la paroi (5) sont disposés sur un substrat (7) en forme de plaque, plan et au moins approximativement lisse, et que les chambres miniatures ou viviers sont définis ou formés par une structure sandwich composée de plaques, de feuilles ou de parois congrues accordées ou adaptées les unes aux autres, l'une au moins de ces plaques, feuilles ou parois présentant, pour former les chambres miniatures ou viviers, une structure alvéolée ou en forme de matrice pourvue de trous (17, 18).

2. Dispositif selon la revendication 1, caractérisé en ce que le substrat est formé par une mince plaque plane parallèle, de préférence lisse, de qualité optique élevée et uniforme.

3. Dispositif selon la revendication 1 ou 2, caractérisé en ce qu'une partie au moins des chambres miniatures ou viviers (17) est formée par une structure ou matrice réticulaire alvéolée (6) qui est posée sur un substrat (7) et qui est solidaire de celui-ci, les ouvertures des chambres miniatures (17) ou des espaces intermédiaires qui sont opposées au substrat étant aptes à être couvertes ou fermées à l'aide d'une membrane de recouvrement (5) ou d'une feuille semi-perméable, laquelle membrane forme la paroi d'une perméabilité apte à être choisie.

4. Dispositif selon l'une des revendications 1 à 3, caractérisé en ce que les chambres miniatures ou viviers individuels (17) sont ronds, avec un diamètre d'environ 0,2 à 5 mm, et en ce qu'une partie au moins des chambres miniatures ou viviers est disposée en forme de structure alvéolée.

5. Dispositif selon l'une des revendications 1 à 4, caractérisé en ce que le substrat (7) est couvert à l'aide d'une couche de gel ou de polymère transparente sans affinité pour les cellules, sur laquelle sont disposés les chambres miniatures ou viviers (17).

6. Dispositif selon l'une des revendications 1 à 5, caractérisé en ce qu'il est prévu sur le fond de chaque chambre, posée sur le substrat ou sur la couche de gel ou de polymère, une tache en forme de point ou d'îlot (18) composée d'une mince couche appropriée pour la culture de cellules.

7. Dispositif selon l'une des revendications 1 à 6, caractérisé en ce que la structure réticulaire ou les parois de chambres (6) se composent d'un matériau rigide et de dimensions stables tel que du polycarbonate, du silicium, un matériau métallique, de la céramique ou un matériau composite.

8. Dispositif selon l'une des revendications 1 à 7, caractérisé en ce que la membrane (5) de préférence semi-perméable se compose d'un matériau polymère tel que du téflon, du polystyrol ou du polycarbonate, présentant des propriétés de perméabilité aptes à être choisies.

9. Dispositif selon l'une des revendications 1 à 8, caractérisé en ce que la plaque ronde a un diamètre d'environ 5 à 20 cm, avec un trou central pour disposer la plaque sur un support de microscope, d'appareil de mesure ou d'appareil de traitement.

10. Dispositif selon l'une des revendications 1 à 9, caractérisé en ce que le dispositif ou la structure est assemblé en sandwich à l'aide d'un dispositif magnétique.

11. Dispositif selon l'une des revendications 1 à 10, caractérisé en ce qu'il est placé dans un récipient (11) tel qu'un boîtier en acier spécial, qui présente d'un côté ou des deux côtés des ouvertures laissant passer la lumière, afin d'effectuer des mesures quand le dispositif se trouve dans le récipient.

12. Procédé pour fabriquer un dispositif pour cultiver des cellules ou d'autres échantillons biologiques, caractérisé par les phases suivantes :
- on forme une structure réticulaire ou alvéolée (6) qui contient comme espaces intermédiaires ou comme trous les chambres miniatures ou viviers ouverts servant d'espaces de culture pour les cellules ou échantillons biologiques individuels, c'est-à-dire pour former des laboratoires miniatures (17),
- on pose et on relie la structure réticulaire ou alvéolée sur une feuille semi-perméable (5) ou membrane de recouvrement,
- après avoir rempli les chambres miniatures ou viviers individuels (17), fermés en bas par la feuille ou membrane de recouvrement semi-perméable, avec des substances nutritives, des sérums, des substances actives ou d'autres composants liquides ou dissous ou solubles, et après avoir placé les cellules (23) ou échantillons biologiques dans les chambres miniatures ou viviers individuels (17), on pose une plaque (7), comme substrat, pour fermer les chambres miniatures ou viviers à l'opposé de la feuille ou membrane de recouvrement semi-perméable (5), et
- on tourne le dispositif formé de 180°.

13. Procédé selon la revendication 12, caractérisé en ce que la paroi semi-perméable est reliée fermement à la structure alvéolée ou réticulaire à l'aide d'un dérivé de silicone ou d'un matériau durcissant aux UV.

14. Procédé selon la revendication 12 ou 13, caractérisé en ce que le substrat en forme de plaque (7) est fabriqué en silicium, en quartz ou en verre, est ensuite revêtu d'une couche transparente de gel ou de polymère sans affinité pour les cellules telle qu'une couche d'agarose, et est enfin pourvu de taches en forme de points ou d'îlots (18) ayant une affinité pour les cellules, lesquelles taches coïncident géométriquement avec les espaces intermédiaires ou les trous (17) de la structure (6), après quoi le substrat est relié de manière coïncidente à la structure de telle sorte que chaque tache vienne se placer globalement au centre de l'espace intermédiaire ou du trou.

15. Procédé selon l'une des revendications 12 à 14, caractérisé en ce que le substrat et la structure sont reliés fermement à l'aide d'une substance adipeuse telle qu'une graisse de silicone.

16. Procédé selon l'une des revendications 12 à 14, caractérisé en ce que la membrane de recouvrement (5) est posée sur une plaque ou une feuille de métal pourvue de trous, les trous correspondant géométriquement aux espaces intermédiaires ou aux trous de la structure, et après avoir posé la structure (6) et le substrat (7), on pose un aimant sur le substrat, à l'opposé de la structure, pour assembler le dispositif grâce à l'attraction exercée par l'aimant sur la plaque ou sur la feuille de métal, et on tourne finalement le dispositif de 180°.

17. Utilisation du dispositif selon l'une des revendications 1 à 11 pour effectuer des mesures, caractérisée en ce que le dispositif ou la structure à chambres est démonté de telle sorte que le matériau cultivé soit libéré et puisse être tué, après quoi des mesures sont effectuées à l'aide d'un rayonnement photonique ou de neutrons sur le matériau tué.

18. Utilisation du dispositif selon l'une des revendications 1 à 11 pour effectuer des mesures, caractérisée en ce que le dispositif ou la structure à chambres est démonté de telle sorte que le matériau cultivé soit libéré pour des méthodes d'analyse biochimiques ou de biologie moléculaire telles que l'électrophorèse ou d'autres méthodes de séparation.
